# EUROPEAN PATENT APPLICATION

(11) **EP 0 654 286 A1**
(43) Date of publication of application: **24.05.1995**
(21) Application number: 94916991.6
(22) Date of filing: 03.06.1994
(51) Int. Cl.: A61N 5/01

(54) **METHOD FOR TREATING VIRAL DISEASES**

(30) Priority: 04.06.1993 ES 9301238
(71) Applicant: ELECTROMECANICA BEKAL, S.L., E-07014 Palma de Mallorca (ES)
(72) Inventor: Konjevic Lisac, Bozidar, E-46014 Torrente (Valencia) (ES)
(74) Representative: Carpintero Lopez, Francisco
(86) International application number: ES9400055
(87) International publication number: WO9428970

(57) **Abstract**

The method consists in interrupting the blood circulation in an area of the body to be treated, preferably in one arm, by means of a tourniquet and, in this situation of temporary interruption of the blood circulation, proceeding to a thermal increase of the latter by application of electromagnetic waves (microwaves), laser or capacitive transmission, up to reaching a temperature capable of destroying the viruses, while providing simultaneously for a continuous agitation of the blood, through percussion or impingement, in order to avoid the coagulation of the blood. The equipment comprises an operative head (12) provided with means for regulating its position as to the height and the orientation so as to adapt the equipment to the patient, the head being provided with a bell (13) for application of heat and vibrations, said bell having a wave emission device which is associated by means of a transmission line (21) to the corresponding generator, whereas the purcusive motion is generated by a motor (11) which, through an excentric (19), imparts an alternate motion against a spring (20) to a percussion piston terminated by an impinger (25).

## Description

### OBJECT OF THE INVENTION

The subject-matter of the present invention relates to a new method for treating virus diseases in general based upon the part-destruction of such viruses whereupon the immunological system of the patient will be in a position to provide a rapid and appropriate response, by creating specific antibodies, to eliminate the rest of the viruses that shall have invaded the body of the patient.

The invention also relates to an electromechanical apparatus for putting into practice the said method.

### BACKGROUND OF THE INVENTION

There is no doubt that viruses causing influenza affect the human population most and there are manifold varieties of these viruses against which specific vaccines are prepared that tend to prevent possible epidemics.

In particular, the said vaccines are manufactured within a laboratory using attenuated viruses that are per se incapable of developing the disease albeit stimulating organic immunology to create specific antibodies to these viruses, and so organism has suitable defences against potential influenza infection.

And yet viruses other than the expected viruses often appear, which is extremely frequent given the extent to which viruses are capable of genetic mutation when they are in an environment that is unfavourable for their normal development and proliferation.

This means that it is almost impossible to prepare a specific vaccine capable of battling against all viruses, having regard to their potential states of genetic mutation, and moreover it is not possible to obtain specific vaccines for certain particular types of viruses, as with the HIV virus causing the Acquired Immune Deficiency Syndrome (AIDS). In all of these cases where vaccine-based preventive treatment is impossible there are frequently endemics or epidemics that have a high social cost because of leaves off work and the consumption of chemotherapeutic products, that at times do not yield the desired results either.

It is also a known fact that the first defence the organism stages against an infection, be it viral, bacterial or originating in another pathogenous germ, comprises raising the body temperature in order to attenuate or destroy the viruses or germs and thereby put into place an appropriate response from the immunological system to create specific antibodies.

The human organism relies on its own temperature adjustment system that maintains blood temperature, regardless of room temperature, at around 37°C.

In the event of a virus infection, for instance influenza, the organism raises this temperature to permissible body levels of around 39-40°C.

There are however other types of viruses that must be subjected to higher temperatures in order to be inactivated or destroyed. Such is the case of the HIV virus aforesaid, which requires a temperature of 56°C to such end.

The human organism has no means capable of raising the blood temperature to this extent and furthermore if the blood were to be heated "in vitro" outside the organism it would be coagulated and hence destroyed.

### DESCRIPTION OF THE INVENTION

The virus disease treatment method subject of the invention has been particularly devised to allow action to be taken in the above sense, viz. allowing blood temperature to be raised to the extent required for the virus cells to be destroyed, without the blood being coagulated nor any side-effect occurring therein, other than fostering the creation of specific antibodies by the immunological bodily system.

More specifically, the method subject hereof makes first of all the blood flow be cut off in a particular area of the body that is to be provided with anti-virus treatment, preferably one of the extremities, and in particular an arm, applying a tourniquet, the purpose being to temporarily isolate the blood mass contained in such member from the rest of the circulatory system, and consequently from the temperature adjustment system that maintains blood temperature at 37°C.

Thereupon the blood in such isolated area is heated, in particular raising the temperature to a suitable level to eliminate the viruses, for instance a temperature of 56°C or higher, using to this end electromagnetic waves (microwaves), laser, capacitive transmission or any other suitable means.

While the temperature is being raised, the blood is permanently agitated to prevent its coagulation, such agitation being intended to achieve an effect equivalent to circulation, which allows the blood to be held back long enough to be able to reach the temperature aforesaid and consequently inactivate or destroy the viruses.

The electromechanical apparatus for putting into practice the said method has a frame or fixed structure upon which a duly powered mobile structure is mounted that is fitted with a support for an operative head, the support having means allowing the said operative head to be trained in any direction in order to be easily adaptable to the body of the patient, the said operative head having a bell member within which a wave emitter is fitted to serve as a heat generator, together with a tapper driven by a percussion motor through a cam that is responsible for transforming the rotary movement of the motor into a reciprocating or percussion movement of the rod or tapper.

### DESCRIPTION OF THE DRAWINGS

In order to provide a fuller description and contribute to the complete understanding of the characteristics of this invention, a set of drawings is attached to the specification which, while purely illustrative and not fully comprehensive, shows the following:
Figure 1.- Is a perspective view of the electromechanical apparatus used for putting into practice the method for treating virus diseases, being the object of the present invention.

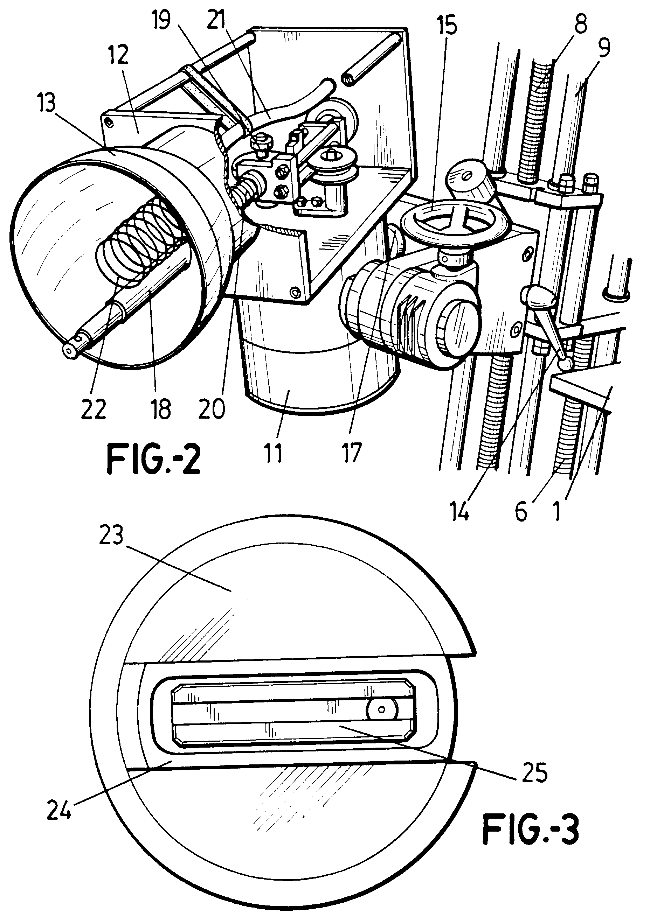

Figure 2.- Is an enlarged close and perspective view of the portion of the apparatus aforesaid fitted with the operative head, showing in particular the bell member, with the cover off to show the inner structure thereof.
Figure 3.- Is a close perspective front view of the operative head bell member, now provided with the respective cover.

### PREFERRED EMBODIMENT OF THE INVENTION

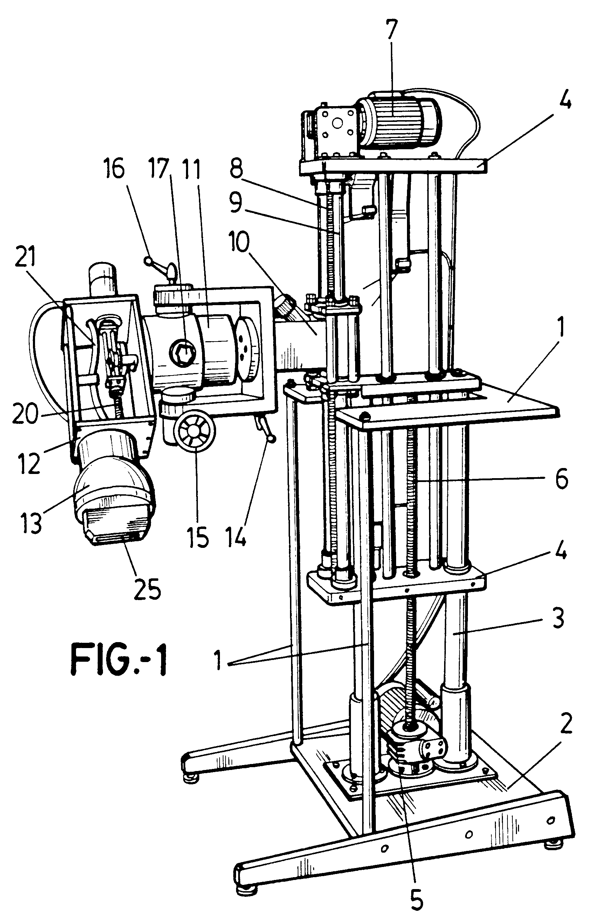

With reference to these figures and in particular figure 1, the apparatus subject hereof actually comprises a fixed frame (1) with vertical slides (3) upstanding from its base platform (2) for a mobile frame (4) to travel, in particular assisted by a motor (5), mounted upon the same base platform (2), that drives a spindle (6) through a transmission for the spindle to provide the mobile frame (4) with vertical travel along the slides (3), the top platform of this mobile frame (4) carrying another motor (7) that also provides vertical travel along slides (9) through a respective reduction unit and a spindle (8) to an operative head support (10), which support is fork-shaped, as shown in particular in figure 1, and has a percussion motor (11) lying between its side branches that is responsible for providing the movement required for the blood in the body area under treatment to be permanently agitated.

More specifically, the body itself of the motor (11) acts as a means joining the head (12) and the support (10), which head (12) is in turn provided with a bell member (13) over it that is the means with which the apparatus is directly applied on the body of the patient, and which may be trained in any direction due to an articulated assembly of such elements that is fixable by means of controls, namely a control (14) that locks the circular rotation of the support (11), a control or turning wheel (15) for the percussion motor (11) between the arms of the support (10), a control (16) fixing the percussion motor, and a tightening control (17) to turn the percussion rod chassis (18) relative to the motor (11).

In particular, movement is transmitted from the motor (11) to the percussion motor or rod (18) through an eccentric (19) mounted on the head (12) and causing a tapper (18) to move against a spring (20) bias.

The head is also provided with a tube (21) for connecting the wave generator, not shown in the figures, to a respective wave emitter (22) located within the bell member (13), that is closed at the front by a cover (23) having a diametric neck (24) through which a tapper (25) linked to the percussion rod (18) projects slightly, and which has a rubber slot or the like, being the element that will contact with the body.

It need only be finally said that the percussion movement generated by the motor (11) must be adjustable, and that this mechanical solution is merely illustrative, for pneumatic type percussion can also be provided.

Additionally, the tapping position can also be automatically and adjustably changed, just as the heat power generated by the emitter (22) can be adjusted.

In accordance with this structure and as aforesaid, the treatment relies upon the destruction of all the viruses there may be in the blood of a body area, raising the temperature therein and thereafter allowing the immunological bodily system to act to cure the disease.

A sphygmomanometer or other instrument measuring blood pressure must be used to separate the area to be treated from the rest of the circulatory system. It is essential to use the same in measuring pulse and be able to adjust blood pressure in order to suitably paralyse blood flow.

Analytical tests made have established that there are no haemolytic effects nor are coagulation factors altered, nor indeed do other side-effects occur, in the blood retained and heated to 56°C, whereas the presence of the HIV antigen is negatived.

An effective therapy is therefore made available to destroy a very wide range of viruses, inter alia HIV, in all their genetic variants, within the human organism, providing the same with all the ingredients in order for such organism to suitable activate its immunological system and consequently provoke its self-immunity.

We feel that the description need not be extended any longer for any expert in the art to have grasped the full scope of the invention and the advantages it offers.

The materials, shape, size and layout of the elements may be altered provided that this entails no modification of the essential features of the invention.

The terms used to describe the invention herein should be taken to have a broad rather than a restrictive meaning.

## Claims

1. Virus desease treatment method, characterised for it consists in the blood flow be cut off in a particular area of the body to be treated, preferably on one of its extremities, such as an arm, applying a tourniquet, subsequently proceeding to raise the blood mass temperature contained at the area to be treated, by means of direct heat application at the organism bosom, through electromagnetic waves, laser, capacitive transmission or any other suitable mean, up to reach the necessary temperature for the virus cells be destroyed, of aroung 56ºC, it having been foreseen that parallel to this temperature raising, a blood permanent agitation be produced in order to prevent its coagulation by means of applying a vibrating movement ot the treated area, produced by a knocking.

2. Method, as in claim 1, characterised in that the percussion or vibration effect is adjustable either in intensity as well as in orientation, the applied heat-power being also adjustable, it further having been foreseen that during the process an arterial pressure control be effected to allow to secure the treatment area isolation with respect to the rest of the circulatory system.

3. An electromechanical virus disease treatment apparatus, characterised by comprising a fixed frame (1) in which a mobile frame (4) can travel vertically, the latter having a support (10) that can travel in the same direction and carries a head (12) that can be trained in any direction, fitted with a bell member (13) for adaption to the part of the body of the patient to be treated, which bell member (13) is internally fitted with a percussion rod (18) that is responsible for generating permanent blood agitation movement, and a wave emitter (22) that is in turn responsible for generating the heat required for the blood temperature to be raised to the required extent.

4. An apparatus as in claim 1, characterised in that the fixed frame (1) has vertical slides (3) on its base platform (2) along which the mobile frame (4) travels, the latter travelling by means of a spindle (6) that is driven by a motor (5), the mobile frame (4) being also fitted with another motor (7) driving a spindle (8) that is responsible for supplying the head support (10) with vertical travel along slides (9).

5. An apparatus as in claims 1 and 2, characterised in that the head support (10) is fork-shaped, and lying between its branches there is a motor (11) responsible for driving the percussion rod (18), said support (10) being fitted with a control (14) to locks its own turning movement, and the motor (11) having a turning wheel (15) between the arms of the support (10), a control (16) for said motor to be fixed and another tightening control (17) to turn the said percussion rod (18) chassis relative to the motor (11).

6. An apparatus as in claims 1, 2 and 3, characterised in that the head (12) is provided with a cam or eccentric (19) through which the rotary movement of the motor (11) is transmitted as a reciprocating movement to the percussion rod (18) against a spring (20), the said head (12) also having a tube (21) connecting the wave emitter (22) lying in the bell member and the respective apparatus generating such waves, and the percussion rod (18) ending in a tapper (24) designed to contact with the body of the patient and which crosses a front cover (23) that closes the said bell member (13).
